(19) Europäisches Patentamt
European Patent Office
Office européen des brevets

(11) Veröffentlichungsnummer: **0 470 430 A1**

# (12) EUROPÄISCHE PATENTANMELDUNG

(21) Anmeldenummer: **91112301.6**

(22) Anmeldetag: **23.07.91**

(51) Int. Cl.[5]: **C07D 239/42**, C07D 239/52, C07D 251/46

(30) Priorität: **04.08.90 DE 4024867**

(43) Veröffentlichungstag der Anmeldung: **12.02.92 Patentblatt 92/07**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Lantzsch, Reinhard Dr.**
**Am Buschhäuschen 51**
**W-5600 Wuppertal(DE)**
Erfinder: **Merz, Walter Dr.**
**Mülhausener Strasse 14**
**W-5090 Leverkusen(DE)**
Erfinder: **Müller, Klaus-Helmut Dr.**
**Bockhackstrasse 55**
**W-4000 Düsseldorf 13(DE)**

(54) **Verfahren zur Herstellung von Oxyguanidinen.**

(57) Die Erfindung betrifft ein Verfahren zur Herstellung von Oxyguanidinen der Formel (I)

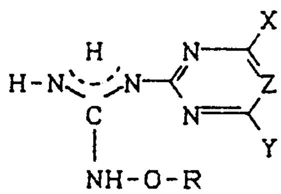

(I)

in welcher

- R für einen jeweils gegebenenfalls substituierten Rest aus der Reihe Alkyl, Alkenyl, Alkinyl, Cycloalkyl, Cycloalkylalkyl, Arylalkyl und Aryl steht,
- X für Wasserstoff oder für jeweils gegebenenfalls substituiertes Alkyl oder Alkoxy steht,
- Y für jeweils gegebenenfalls substituiertes Alkyl oder Alkoxy steht und
- Z für Stickstoff oder eine CH-Gruppierung steht.

Man erhält die Verbindungen (I) (welche als Zwischenprodukte zur Herstellung von bestimmten Herbiziden verwendet werden können) in guten Ausbeuten, indem man Isoharnstoffe der allgemeinen Formel (II)

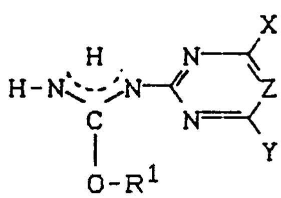

(II)

in welcher

EP 0 470 430 A1

$R^1$ für jeweils gegebenenfalls substituiertes Alkyl oder Arylalkyl steht,
mit Hydroxylaminderivaten der allgemeinen Formel (III)

$H_2N$-O-R (III)

oder mit Hydrogenhalogeniden von Verbindungen der Formel (III) gegebenenfalls in Gegenwart eines Säureakzeptors und gegebenenfalls in Gegenwart eines Verdünnungsmittels bei Temperaturen zwischen -20°C und +150°C umsetzt.

Die Erfindung betrifft ein neues Verfahren zur Herstellung von bekannten Oxyguanidinen.

Es ist bekannt, daß man Oxyguanidine erhält, wenn man Cyanaminoverbindungen mit Hydroxylaminderivaten umsetzt (vgl. J. Chem. Soc. C 1966, 2031-2038; EP-A 121 082/US-P 4 602 938).

Diese Herstellungsmethode hat den Nachteil, daß hierbei Hydroxylaminderivate, die zum Teil relativ teuer sind, in großem Überschuß (im allgemeinen zwei bis drei Moläquivalente) zum Erzielen von guten Ausbeuten eingesetzt werden müssen.

Weiter ist bekannt, daß Oxyguanidine auch durch Umsetzung von Isothioharnstoffen mit Hydroxylaminderivaten hergestellt werden können (vgl. EP-A 358018). Auch bei diesem Verfahren werden die Hydroxylaminderivate im allgemeinen im Überschuß eingesetzt. Außerdem werden giftige und übelriechende Mercaptoverbindungen freigesetzt, deren Entsorgung bei einer großtechnischen Produktion sehr aufwendig ist. Die als Ausgangsstoffe benötigten Isothioharnstoffe werden zudem unter Verwendung des sicherheitstechnisch sehr problematischen Schwefelkohlenstoffs hergestellt.

Es wurde nun gefunden, daß man Oxyguanidine der allgemeinen Formel (I)

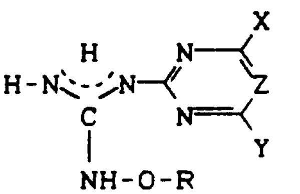

(I)

in welcher

R für einen jeweils gegebenenfalls substituierten Rest aus der Reihe Alkyl, Alkenyl, Alkinyl, Cycloalkyl, Cycloalkylalkyl, Arylalkyl und Aryl steht,

X für Wasserstoff oder für jeweils gegebenenfalls substituiertes Alkyl oder Alkoxy steht,

Y für jeweils gegebenenfalls substituiertes Alkyl oder Alkoxy steht und

Z für Stickstoff oder eine CH-Gruppierung steht,

in guten Ausbeuten und in hoher Reinheit erhält, wenn man Isoharnstoffe der allgemeinen Formel (II)

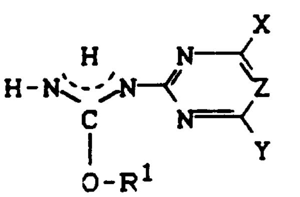

(II)

in welcher

X, Y und Z die oben angegebenen Bedeutungen haben und

$R^1$ für jeweils gegebenenfalls substituiertes Alkyl oder Arylalkyl steht,

mit Hydroxylaminderivaten der allgemeinen Formel (III)

$H_2N\text{-}O\text{-}R$ (III)

in welcher

R die oben angegebene Bedeutung hat,

- oder mit Hydrogenhalogeniden von Verbindungen der Formel (III) -

gegebenenfalls in Gegenwart eines Säureakzeptors und gegebenenfalls in Gegenwart eines Verdünnungsmittels bei Temperaturen zwischen -20 °C und +150 °C umsetzt.

Überraschenderweise können nach dem erfindungsgemäßen Verfahren die Verbindungen der Formel (I) auf relativ einfache Weise in hohen Ausbeuten hergestellt werden, wobei die Nachteile der bekannten Verfahren - großer Überschuß eines Ausgangsproduktes bzw. Anfall problematischer Abspaltungsprodukte - vermieden werden.

Verwendet man beispielsweise N-(4,6-Dimethoxypyrimidin-2-yl)-O-methyl-isoharnstoff und O-Methyl-hydroxylamin als Ausgangsstoffe, so kann der Reaktionsablauf durch das folgende Formelschema skizziert werden:

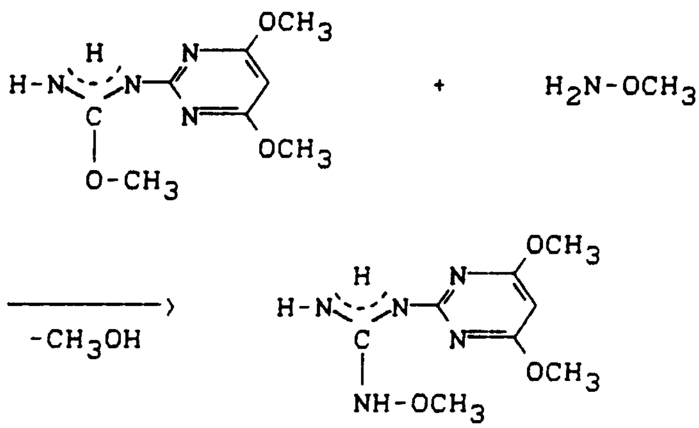


Die als Ausgangsstoffe zu verwendenden Isoharnstoffe sind durch die Formel (II) allgemein definiert. In Formel (II) stehen vorzugsweise

- $R^1$ für $C_1$-$C_4$-Alkyl (welches gegebenenfalls durch Cyano, Carboxy oder $C_1$-$C_2$-Alkoxy-carbonyl substituiert ist) oder für Benzyl (welches gegebenenfalls durch Halogen und/oder $C_1$-$C_2$-Alkyl substituiert ist),
- X für Wasserstoff, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy oder $C_1$-$C_4$-Halogenalkoxy,
- Y für $C_1$-$C_4$-Alkyl, $C_1$-$C_4$-Halogenalkyl, $C_1$-$C_4$-Alkoxy oder $C_1$-$C_4$-Halogenalkoxy, und
- Z für Stickstoff oder eine CH-Gruppierung.

Als Ausgangsstoffe besonders bevorzugt werden die Verbindungen der Formel (II), in welcher

- $R^1$ für Methyl, Ethyl, Carboxymethyl, Methoxycarbonylmethyl, Ethoxycarbonylmethyl oder Benzyl - insbesondere für Methyl - steht,
- X für Wasserstoff, Methyl, Ethyl, Trifluormethyl, Methoxymethyl, Methoxy, Ethoxy oder Difluormethoxy - insbesondere für Methyl oder Methoxy - steht,
- Y für Methyl, Ethyl, Trifluormethyl, Methoxy, Ethoxy oder Difluormethoxy - insbesondere für Methyl oder Methoxy - steht, und
- Z für Stickstoff oder eine CH-Gruppierung - insbesondere für eine CH-Gruppierung - steht.

Als Beispiele für die Ausgangsstoffe der Formel (II) seien genannt:
N-(4,6-Dimethyl-pyrimidin-2-yl)-, N-(4,6-Dimethoxy-pyrimidin-2-yl)-, N-(4,6-Diethoxy-pyrimidin-2-yl)-, N-(4-Methoxy-6-methyl-pyrimidin-2-yl)-, N-(4-Ethoxy-6-methyl-pyrimidin-2-yl)-, N-(4-Ethyl-6-methoxy-pyrimidin-2-yl)-, N-(4-Methoxy-6-trifluormethyl-pyrimidin-2-yl)-, N-(4,6-Bis-trifluormethyl-pyrimidin-2-yl)- und N-(4,6-Bis-difluormethoxy-pyrimidin-2-yl)-O-methyl-isoharnstoff.

Die Isoharnstoffe der Formel (II) sind bekannt oder Gegenstand einer nicht vorveröffentlichten Patentanmeldung (vgl. Chem. Pharm. Bull. 21 (1973), 478-482; DE-P 3927788 vom 23.08.1989; Herstellungsbeispiele).

Die beim erfindungsgemäßen Verfahren zur Herstellung der Oxyguanidine der Formel (I) weiter als Ausgangsstoffe zu verwendenden Hydroxylaminderivate sind durch die Formel (III) allgemein definiert. In Formel (III) steht vorzugsweise

- R für $C_1$-$C_{12}$-Alkyl (welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, Cyano oder Nitro substituiert ist), $C_3$-$C_6$-Alkenyl (welches gegebenenfalls durch Fluor oder Chlor substituiert ist), $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)-amino-carbonyl-$C_1$-$C_2$-alkyl, für Phenyl, Phenylethyl, Benzhydryl oder Benzyl (welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxycarbonyl substituiert sind).

Als Ausgangsstoffe besonders bevorzugt werden die Verbindungen der Formel (III), in welcher

- R für $C_1$-$C_5$-Alkyl, $C_3$-$C_5$-Alkenyl, $C_1$-$C_3$-Alkoxy-carbonyl-$C_1$-$C_2$-alkyl oder Benzyl (welches gegebenenfalls durch Fluor, Chlor, Methyl, Ethyl, Methoxy, Ethoxy, Methoxycarbonyl und/oder Ethoxycarbonyl substituiert ist) - insbesondere für Methyl - steht.

Als Beispiele für die Ausgangsstoffe der Formel (III) seien genannt:
O-Methyl-hydroxylamin, O-Ethyl-hydroxylamin, O-Propyl-hydroxylamin, O-Isopropyl-hydroxylamin, O-Butylhydroxylamin, O-Isobutyl-hydroxylamin, O-Pentyl-hydroxylamin, O-Allyl-hydroxylamin, O-Methoxycarbonylmethyl-hydroxylamin, O-Ethoxycarbonylmethyl-hydroxylamin, O-Benzyl-hydroxylamin, O-(4-Chlor-benzyl)-hydroxylamin, O-(4-Fluor-benzyl)-hydroxylamin, O-(4-Methyl-benzyl)-hydroxylamin, O-(4-Methoxy-carbonyl-benzyl)-hydroxylamin und O-(4-Ethoxycarbonyl-benzyl)-hydroxylamin.

Die Hydroxylaminderivate der Formel (III) sind bekannt und/oder können nach an sich bekannten Verfahren hergestellt werden (vgl. Chem. Pharm. Bull. 15 (1967), 345; Synthesis 1976, 682; J. Chem. Soc. 1930, 228; Helv. Chim. Acta 45 (1962), 1387).

Das erfindungsgemäße Verfahren zur Herstellung der Oxyguanidine der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, Propanol, Isopropanol, Butanol, Isobutanol, sec-Butanol und tert-Butanol, Ketone wie Aceton, Methyl-ethyl-, Methyl-isopropyl- und Methylisobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Alkohole, wie insbesondere Methanol, Ethanol und Isopropanol, werden als Verdünnungsmittel ganz besonders bevorzugt.

Als Säureakzeptoren können bei dem erfindungsgemäßen Verfahren alle üblicherweise für derartige Umsetzungen verwendbaren Säurebindemittel eingesetzt werden. Vorzugsweise infrage kommen Alkalimetall- und Erdalkalimetall-hydride, wie Lithium-, Natrium-, Kalium- und Calcium-hydrid, Alkalimetall- und Erdalkalimotall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und -tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethyl-benzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Das erfindungsgemäße Verfahren wird im allgemeinen bei Temperaturen zwischen -20 °C und +150 °C, vorzugsweise zwischen 0 °C und 100 °C, und im allgemeinen bei Normaldruck durchgeführt.

Zur Durchführung des erfindungsgemäßen Verfahrens setzt man je Mol Isoharnstoff der Formel (II) im allgemeinen zwischen 1 und 2 Mol, vorzugsweise zwischen 1,1 und 1,5 Mol, Hydroxylaminderivat der Formel (III) ein.

Es können auch entsprechende Mengen von Hydrogenhalogeniden - insbesondere von Hydrochloriden - der Verbindungen der Formel (III) eingesetzt werden.

Die Ausgangsstoffe können in beliebiger Reihenfolge zusammengegeben werden. Das Reaktionsgemisch wird bei der jeweils erforderlichen Temperatur bis zum Ende der Umsetzung gerührt.

Die Aufarbeitung kann nach üblichen Methoden durchgeführt werden. Im allgemeinen wird eingeengt, der Rückstand in einem mit Wasser praktisch nicht mischbaren organischen Lösungsmittel, wie z.B. Methylenchlorid, aufgenommen, diese Lösung mit Wasser gewaschen, getrocknet und filtriert. Vom Filtrat wird das Lösungsmittel unter vermindertem Druck sorgfältig abdestilliert, wobei das Produkt der Formel (I) als kristalliner oder amorpher Rückstand verbleibt.

Die nach dem erfindungsgemäßen Verfahren herzustellenden Oxyguanidine der Formel (I) können als Zwischenprodukte zur Herstellung von Herbiziden verwendet werden (vgl. EP-A 121 082).

Herstellungsbeispiele:

Beispiel 1

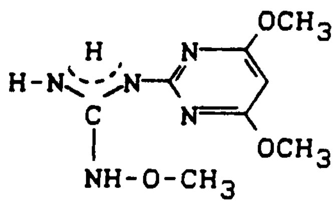

Eine Mischung aus 10,9 g (0,13 Mol) O-Methylhydroxylammoniumchlorid, 10,7 g (0,13 Mol) Natriumacetat und 200 ml Methanol wird bei 20 °C unter Rühren mit 21,2 g (0,10 Mol) N-(4,6-Dimethoxy-pyrimidin-2-yl)-O-methyl-isoharnstoff versetzt und das Reaktionsgemisch wird 16 Stunden bei 20 °C gerührt. Dann wird eingeengt, der Rückstand in Methylenchlorid aufgenommen, mit Wasser, 5%iger Natriumhydrogencarbonatlösung und erneut mit Wasser gewaschen, mit Natriumsulfat getrocknet und filtriert. Vom Filtrat wird das Lösungsmittel im Wasserstrahlvakuum sorgfältig abdestilliert.

Man erhält 19,7 g (87% der Theorie) N'-Methoxy-N''-(4,6-dimethoxy-pyrimidin-2-yl)-guanidin als kristallinen Rückstand vom Schmelzpunkt 122 °C.

Beispiel 2

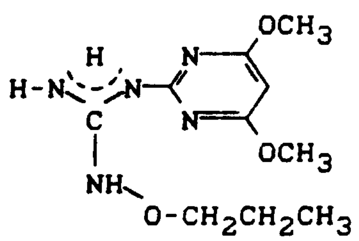

Eine Mischung aus 13,5 g (0,18 Mol) O-Propylhydroxylamin, 32 g (0,15 Mol) N-(4,6-Dimethoxy-pyrimidin-2-yl)-O-methyl-isoharnstoff und 200 ml Methanol wird 24 Stunden unter Rückfluß zum Sieden erhitzt. Dann wird eingeengt, der Rückstand in Methylenchlorid aufgenommen, mit Wasser gewaschen, mit Natriumsulfat getrocknet und filtriert. Vom Filtrat wird das Lösungsmittel im Wasserstrahlvakuum sorgfältig abdestilliert.

Man erhält 34 g (89% der Theorie) N'-Propoxy-N''-(4,6-dimethoxy-pyrimidin-2-yl)-guanidin als öligen Rückstand vom Brechungsindex $n_D^{20}$ = 1,5586.

Analog Beispiel 1 oder 2 wurden erhalten:

Beispiel 3

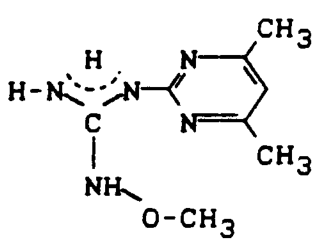

N'-Methoxy-N''-(4,6-dimethyl-pyrimidin-2-yl)-guanidin (Schmelzpunkt: 142 °C; Ausbeute: 81% der Theorie).

Beispiel 4

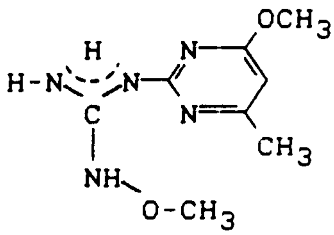

N'-Methoxy-N''-(4-methoxy-6-methyl-pyrimidin-2-yl)-guanidin (Schmelzpunkt: 135 °C; Ausbeute: 73% der Theorie).

Ausgangsstoffe der Formel (II):

Beispiel (II-1)

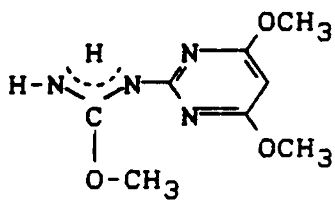

54,5 g (0,25 Mol) 2-Methylsulfonyl-4,6-dimethoxy-pyrimidin, 69 g (0,5 Mol) Kaliumcarbonat und 1500 ml Acetonitril werden unter Rühren vermischt. Dann gibt man 33,85 g (0,1375 Mol) O-Methyl-isoharnstoffsulfat zu und erhitzt 17 Stunden zum Sieden. Nach Abkühlen wird abgesaugt und die Mutterlauge eingeengt. Nach Aufnehmen in Methylenchlorid wäscht man dreimal mit Wasser, trocknet und engt erneut ein.

Man erhält 49,9 g N-(4,6-Dimethoxy-pyrimidin-2-yl)-O-methyl-isoharnstoff in Form gelber Kristalle vom Schmelzpunkt 91-95 °C (Gehalt 90%; Ausbeute: 85% der Theorie).

Beispiel (II-2)

Stufe 1:

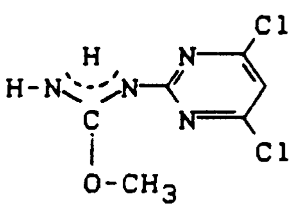

45,9 g (0,25 Mol) 2,4,6-Trichlorpyrimidin, 30,8 g (0,125 Mol) O-Methyl-isoharnstoffsulfat und 51,8 g (0,375 Mol) gemahlenes Kaliumcarbonat werden in 800 ml Toluol suspendiert und 10 Stunden bei 110 °C gerührt. Nach dem Abkühlen wird der kristalline Feststoff abgesaugt und 15 Minuten mit Wasser verrührt und erneut abgesaugt.

Man erhält 39,4 g N-(4,6-Dichlor-pyrimidin-2-yl)-O-methyl-isoharnstoff (GC-Gehalt 98%: 38,6 g; Ausbeute: 69,9% der Theorie), Schmelzpunkt 178 °C.

Stufe 2:

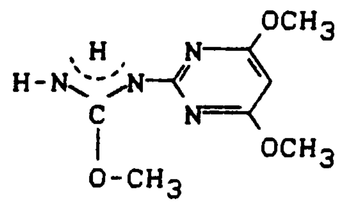

22,1 g (93,8%ig: 0,0938 Mol) N-(4,6-Dichlor-pyrimidin-2-yl)-O-methyl-isoharnstoff werden in 200 ml Methanol suspendiert; dazu wird eine Lösung von 13,5 g (0,25 Mol) Natriummethylat in 100 ml Methanol tropfenweise gegeben. Das Reaktionsgemisch wird 16 Stunden unter Rückfluß erhitzt und dann eingeengt. Der Rückstand wird in Wasser aufgenommen und dreimal mit Methylenchlorid extrahiert. Von den vereinigten Extrakten wird das Lösungsmittel abdestilliert.

Man erhält 19,9 g (100% der Theorie) N-(4,6-Dimethoxy-pyrimidin-2-yl)-O-methyl-isoharnstoff als kristallinen Rückstand vom Schmelzpunkt 98 ° C.

**Patentansprüche**

**1.** Verfahren zur Herstellung von Oxyguanidinen der allgemeinen Formel (I),

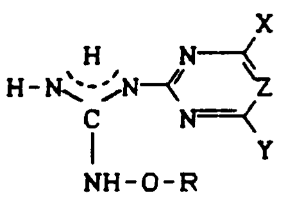

(I)

in welcher

R für einen jeweils gegebenenfalls substituierten Rest aus der Reihe Alkyl, Alkenyl, Alkinyl, Cycloalkyl, Cycloalkylalkyl, Arylalkyl und Aryl steht,

X für Wasserstoff oder für jeweils gegebenenfalls substituiertes Alkyl oder Alkoxy steht,

Y für jeweils gegebenenfalls substituiertes Alkyl oder Alkoxy steht und

Z für Stickstoff oder eine CH-Gruppierung steht,

dadurch gekennzeichnet, daß man Isoharnstoffe der allgemeinen Formel (II)

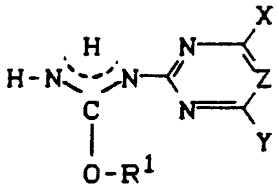

(II)

in welcher

X, Y und Z die oben angegebenen Bedeutungen haben und

$R^1$ für jeweils gegebenenfalls substituiertes Alkyl oder Arylalkyl steht,

mit Hydroxylaminderivaten der allgemeinen Formel (III)

$$H_2N-O-R \quad (III)$$

in welcher

R die oben angegebene Bedeutung hat,

- oder mit Hydrogenhalogeniden von Verbindungen der Formel (III) -

gegebenenfalls in Gegenwart eines Säureakzeptors und gegebenenfalls in Gegenwart eines Verdünnungsmittels bei Temperaturen zwischen -20° C und +150° C umsetzt.

**2.** Verfahren gemäß Anspruch 1, dadurch gekennzeichnet, daß die Umsetzung bei Temperaturen zwischen 0° und 100° C durchgeführt wird.

**3.** Verfahren gemäß Anspruch 1, dadurch gekennzeichnet, daß man Isoharnstoffe der Formel (II) einsetzt, in welcher

$R^1$ für $C_1$-$C_4$-Alkyl (welches gegebenenfalls durch Cyano, Carboxy oder $C_1$-$C_2$-Alkoxy-carbonyl substituiert ist) oder für Benzyl steht (welches gegebenenfalls durch Halogen und/oder $C_1$-$C_2$-Alkyl substituiert ist),

X für Wasserstoff, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy oder $C_1$-$C_4$-Halogenalkoxy steht,

Y für $C_1$-$C_4$-Alkyl, $C_1$-$C_4$-Halogenalkyl, $C_1$-$C_4$-Alkoxy oder $C_1$-$C_4$-Halogenalkoxy steht und

Z für Stickstoff oder eine CH-Gruppierung steht.

**4.** Verfahren gemäß Anspruch 1, dadurch gekennzeichnet, daß man Isoharnstoffe der Formel (II) einsetzt, in welcher

$R^1$ für Methyl, Ethyl, Carboxymethyl, Methoxycarbonylmethyl, Ethoxycarbonylmethyl oder Benzyl steht,

X für Wasserstoff, Methyl, Ethyl, Trifluormethyl, Methoxymethyl, Methoxy, Ethoxy oder Difluormethoxy steht,

Y für Methyl, Ethyl, Trifluormethyl, Methoxy, Ethoxy oder Difluormethoxy steht, und

Z für Stickstoff oder eine CH-Gruppierung steht.

**5.** Verfahren gemäß Anspruch 1, dadurch gekennzeichnet, daß man Hydroxylaminderivate der Formel (III) einsetzt, in welcher

R für $C_1$-$C_{12}$-Alkyl (welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, Cyano oder Nitro substituiert ist), $C_3$-$C_6$-Alkenyl (welches gegebenenfalls durch Fluor oder Chlor substituiert ist), $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)-amino-carbonyl-$C_1$-$C_2$-alkyl, für Phenyl, Phenylethyl, Benzhydryl oder Benzyl (welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxy-carbonyl substituiert sind) steht.

**6.** Verfahren gemäß Anspruch 1, dadurch gekennzeichnet, daß man Hydroxylaminderivate der Formel (III) einsetzt, in welcher

R für $C_1$-$C_5$-Alkyl, $C_3$-$C_5$-Alkenyl, $C_1$-$C_3$-Alkoxy-carbonyl-$C_1$-$C_2$-alkyl oder Benzyl (welches gegebenenfalls durch Fluor, Chlor, Methyl, Ethyl, Methoxy, Ethoxy, Methoxycarbonyl und/oder Ethoxycarbonyl substituiert ist) -insbesondere für Methyl- steht.

Europäisches Patentamt

# EUROPÄISCHER RECHERCHENBERICHT

Nummer der Anmeldung

**EP 91 11 2301**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 358 018 (BAYER AG)<br>* Ansprüche 1,2 *<br>– – – | 1-6 | C 07 D 239/42<br>C 07 D 239/52<br>C 07 D 251/46 |
| Y | EP-A-0 343 462 (BAYER AG)<br>* Anspruch 2 mit Beispielen Seite 19 *<br>– – – | 1-6 | |
| Y | EP-A-0 302 378 (BAYER AG)<br>* Anspruch 4 mit Beispielen Seite 19 *<br>– – – – – | 1-6 | |

**RECHERCHIERTE SACHGEBIETE (Int. Cl.5)**

C 07 D 239/00
C 07 D 251/00
C 07 D 521/00

Der vorliegende Recherchenbericht wurde für alle Patentansprüche erstellt

| Recherchenort | Abschlußdatum der Recherche | Prüfer |
|---|---|---|
| Berlin | 12 November 91 | FRELON D.L.M.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument